Europäisches Patentamt

(19) European Patent Office

Office européen des brevets

(11) Publication number: **0 166 305 B1**

# EUROPEAN PATENT SPECIFICATION

(12)

(45) Date of publication of patent specification: **07.08.91**

(21) Application number: **85107235.5**

(22) Date of filing: **12.06.85**

(51) Int. Cl.⁵: **A61M 16/00, A61M 16/12, A61M 16/01**

(54) Anaesthesia and/or respirator arrangement with alternative manual operation.

<table>
<tr><td>

(30) Priority: **28.06.84 SE 8403450**

(43) Date of publication of application:
**02.01.86 Bulletin 86/01**

(45) Publication of the grant of the patent:
**07.08.91 Bulletin 91/32**

(84) Designated Contracting States:
**AT BE CH DE FR GB IT LI LU NL**

(56) References cited:
**US-A- 3 307 542**
**US-A- 3 467 092**

</td><td>

(73) Proprietor: **GAMBRO ENGSTRÖM AB
Box 201 09
S-161 20 Bromma(SE)**

(72) Inventor: **Broddner, Sven Malte
Vängevägen 33
S-194 51 Upplands Väsby(SE)**
Inventor: **Brömster, Leif
Släggbacken 1
S-171 57 Solna(SE)**
Inventor: **Lundell, Ulf
Bergfinkvägen 27
S-140 32 Grödinge(SE)**
Inventor: **Pilenvik, Göran Sigvard
Karlbergsvägen 23
S-113 27 Stockholm(SE)**

(74) Representative: **Boberg, Nils Gunnar Erik
Gambro AB Patent Department Box 10101
S-220 10 Lund(SE)**

</td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

### TECHNICAL FIELD

The present invention relates to an anaesthesia and/or respirator arrangement comprising a gas preparation section which is adapted to prepare a treatment gas of gas from one or more gas sources, and a mechanically operated ventilator section, by means of which the prepared gas mixture is supplied to the patient, the said ventilator section being adapted so that it also can be operated with the help of a manual reserve system which comprises a manually actuated pump which is adapted to act with the help of a gaseous propellant from the outside upon a movable wall in a chamber containing the treatment gas, the treatment gas and the propellant being completely separated from each other.

The invention is intended primarily to be used as a complete anaesthesia arrangement. However, it will be clear to those versed in the art that in many respects it can also be applied to an arrangement intended merely to be used as a respirator, as many functions are of a similar kind in these two types of arrangements.

### BACKGROUND ART

Known systems frequently suffer the disadvantage of being technically complicated and therefore expensive. Simpler and cheaper systems exist, of course, but they may in such a case be unreliable. If, for example, a manually controlled bag or bellows is connected directly to a duct leading to the patient, this requires a special change-over system. It also makes necessary a cleaning between each treatment. Any leakage, moreover, may contain dangerous gases.

Arrangements of the above kind are described in for instance US-A-3 307 542 and US-A-3 467 092. The preamble of the following claim 1 is therefor based on US-A-3 467 092. It should furthermore be noted that US-A-3 307 542 discloses a changeover valve which, however, has to be manually operated.

### DISCLOSURE OF INVENTION

An arrangement according to the present invention is defined in the following claims.

By means of the present invention an anaesthesia and/or respirator arrangement of the aforementioned type is obtained which is of a simple set-up, whilst the manual operation can be carried out by means of a harmless propellant, e.g. taken directly from one of the gas sources which are used for the preparation of the treatment gas.

More particularly this is achieved in that the manually actuated pump is adapted to act with the help of the gaseous propellant from the outside upon a movable wall in a chamber containing the treatment gas, the treatment gas and the propellant being completely separated from each other.

The chamber containing the treatment gas is acted upon, also during normal mechanical operation, by the pressure of one of the treatment gases, e g air or oxygen. In this case too the gas is taken directly from the source, that is to say before the preparation of the treatment gas. In order to generate a suitable pressure for this purpose a reducing valve is preferably arranged between the said source and the chamber whose movable wall is to be acted on.

In accordance with the invention a change-over valve is provided in the duct between the said source and the said chamber which automatically disconnects the said source when the manually actuated pump is acted on to produce a pressure upon the movable wall.

It will be evident to those versed in the art that the above mentioned chamber may be designed in many different ways. Preferably, however, it is constituted of a bag, bellows or the like, compressible from the outside, which in turn is arranged in a closed rigid chamber.

For reasons of safety the treatment gas and/or the propellant for the same is appropriately arranged so as to be in connection with a safety valve which opens at the maximum permissible pressure.

A safe continuous method of operation is achieved if the said change-over valve is adapted to be reset automatically when the manual operation ceases, so that the chamber containing the treatment gas is subjected once again to normal mechanical operation.

This automatic resetting is particularly valuable if the ventilator section is adapted to be controlled according to the so-called EMMV-method (Extended Mandatory Minute Ventilation) where the inspiratory gas supplied to the patient is continuously measured and integrated and compared with the integral of a corresponding set value. This method is described in more detail for example in the US-A-4 421 113 whose content, therefore, is incorporated in the present description. Thanks to this automatic feedback an operation can be provided wherein the inspiratory gas supplied manually too is measured and integrated, the result obtained being added to the integral of the mechanically supplied or spontaneously inspirated treatment gas. A simple design of this kind can be obtained if the mechanically supplied gas and the manually supplied gas are both measured by means of the same meter downstream of the said change-over valve.

## BRIEF DESCRIPTION OF THE DRAWING

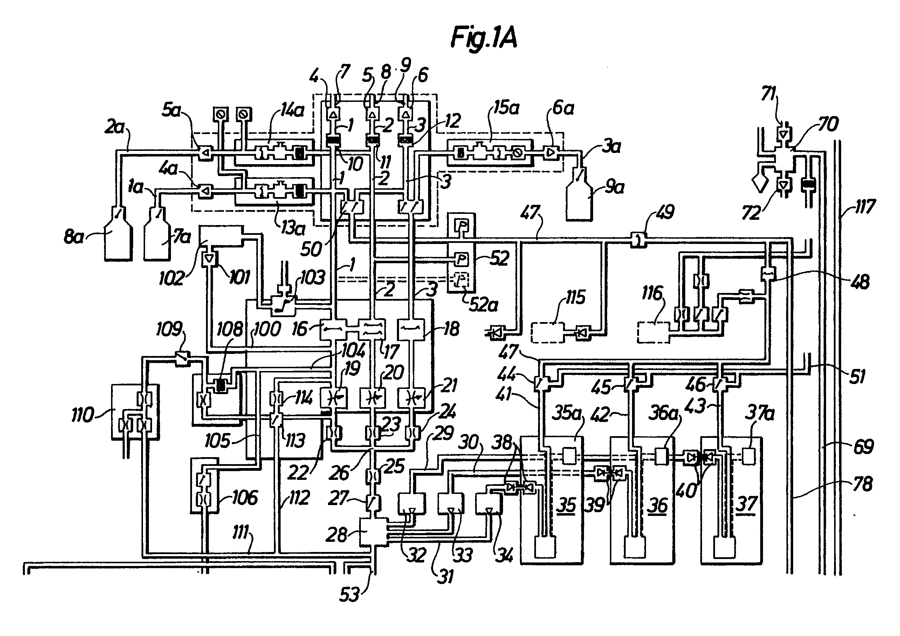

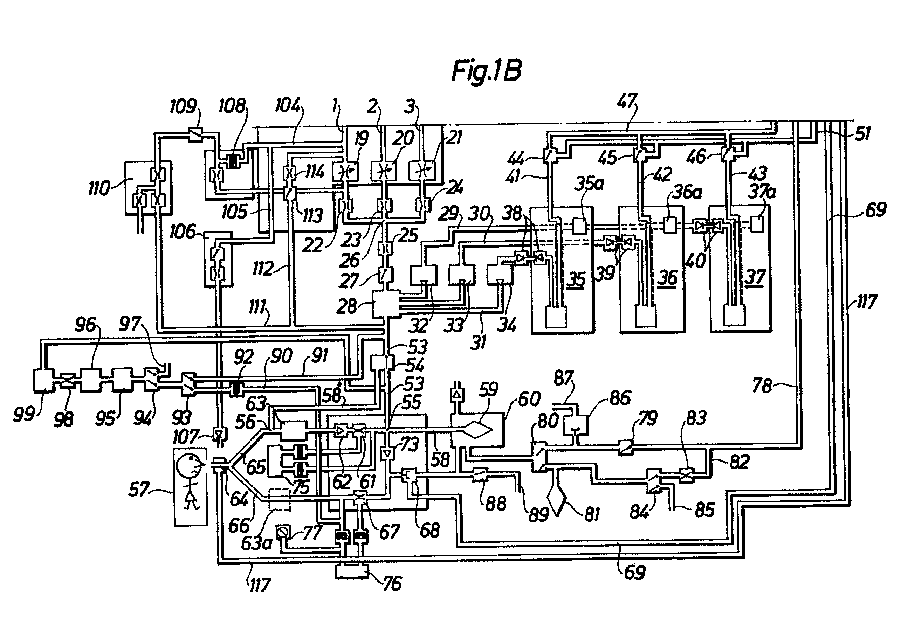

On the enclosed drawing a substantially complete anaesthesia arrangement is described diagrammatically in the form of a block diagram. It should be evident, however, to those versed in the art which components could be omitted if the invention were to be applied to an arrangement merely intended to be used as a respirator. For practical reasons the drawing has been divided into part figures 1A and 1B with certain components being shown in both part figures.

## BEST MODE OF CARRYING OUT THE INVENTION

On the enclosed drawing is thus shown a preferred embodiment of the present invention. Numerals 1, 2 and 3 designate three parallel inlet ducts which via check valves 4, 5 and 6 are connected to attachments 7, 8 and 9 to gas sources (not shown) which may constitute fixed installations in a hospital. For the filtering out of any particles the ducts 1, 2 and 3 contain filters 10, 11 and 12.

Alternatively the ducts 1, 2 and 3 may be connected instead via ducts 1a, 2a and 3a to separate gas sources, e.g. loose gas tubes 7a, 8a and 9a. In this case this is done via check valves 4a, 5a and 6a and the filter and pressure control arrangements shown schematically on the drawing, and designated as a whole by reference numerals 13a, 14a and 15a.

In the system shown it is assumed that oxygen gas is to be supplied from the sources 7 or 7a and laughing-gas $N_2O$ from the sources 8 or 8a. From the source 9 compressed air is supplied and as an alternative carbon dioxide $CO_2$ from the source 9a.

A first pressure control of the gases supplied takes place in the pressure control valves 16, 17 and 18 which are followed by fine control valves, e.g. needle valves 19, 20 and 21. Thereafter the flows obtained are monitored by flow meters 22, 23 and 24 and a further flow meter 25 arranged after the mixing point 26 of the gases. The cross connection between the valves 16 and 17 indicates that the valve 16 is adapted to control the valve 17, so that the gas stream in the duct 2 will be dependent , e.g. proportionally,on that in the duct 1. For practical reasons the drawing has been divided into part figures 1A and 1B with certain components being shown on both part figures.

The gas mixture is then introduced via a valve 27 into a gasifier 28. Liquid is supplied to this gasifier via ducts 29, 30 and 31 comprising valves 32, 33 and 34 from each of three liquid sources 35, 36 and 37 which may contain, for example, halothane, enflurane or isoflurane. These liquid sources may consist of normal glass bottles which can be connected via double valves 38, 39 and 40 directly to the ducts 29, 30 and 31.

Numerals 35a, 36a and 37a designate level gauges for the control of the level in the respective liquid source.

As is evident from the ducts 41, 42 and 43 with the valves 44, 45 and 46 and the duct 47 with the pressure control valves 48 and 49 the liquid sources 35, 36 and 37 are pressurized with the help of gas either from the duct 1, that is to say oxygen gas, or the duct 3, that is to say compressed air. The choice between the two can be made by means of the valve 50.

With the help of the valves 44, 45 and 46 and the duct 51 the liquid sources 35, 36 and 37 can be evacuated when they are not required to be under pressure. The duct 51 is appropriately connected directly to the normal evacuation system of the hospital, e.g. via the collecting valve 70 described more closely in the following.

The pressure in the duct 2 and in the duct 47 respectively after the valve 50 is read appropriately by means of the pressure gauge 52 shown schematically which also may contain a pressure gauge 52a for reading the pressure in the duct 1, as shown by broken lines.

The gasification occurs suitably in such a manner that one of the valves 32, 33 and 34 is opened periodically with a prescribed frequency and over a prescribed length of period. In this manner a prescribed quantity of liquid anaesthetic is fed to the gasifier 28 where in accordance with a preferred embodiment of the subject of the invention it is gasified on a heating surface. By monitoring the energy supply to, and possibly the temperature of, this heating surface a further measure of the quantity of liquid fed is obtained which can thus be checked in two ways. With regard to this function reference is made, moreover, to SE-B-449 565 of the title "Anaesthesia and/or respirator arrangement with a moistening and/or gasification chamber".

From the gasifier 28 the prepared mixed gas is conducted via a duct 53 with a valve 54 either to a first point 55 or a second point 56 in a recirculation system which can be connected to the patient 57. The gag is conducted out to the point 56 via a shunt line 58' if a rapid change of composition of the gas supplied to the patient is desired. Normally, though, it is conducted to the point 55 which through a duct 58 is connected to a bubble or bellows device 59 in a closed housing 60. When the bubble or bellows device 59 is subjected to a pressure, the gas is pressed via a throttle 61, a check valve 62 and possibly a carbon dioxide absorbent 63 via a breathing mask 64 or the like to the patient. The inspiration takes place here via a branch 65 and the expiration via a branch 66. The expiration takes place via a throttle 67 and an expiration valve 68, which via a duct 69 and a safety valve 70 is connected to the evacuation system of the hospital.

Owing to the safety valve 70 being provided with two by-pass valves 71 and 72 the expiratory pressure of the patient can be limited so that it remains within accurately defined limits. For example, the valve 71 can be adapted so that it opens when the pressure is below -1 cm $H_2O$ and the valve 72 opens when the pressure exceeds + 10 cm $H_2O$.

However, normally only a small portion of the expiratory gas is let out via the valve 68. The bulk is recirculated instead via the check valve 73 back to the patient 57. The circulating stream is measured on the one hand over the throttle valve 61, on the other hand over the throttle valve 67. This is done with the help of pressure gauges 75 and 76, shown schematically, which measure the pressure drop over the respective throttle and consequently the flow through the same. For a further check the flow meter 76 is coordinated with a pressure guage 77.

It is indicated by means of broken lines that alternatively the carbon dioxide absorbent 63 can be replaced by an absorbent 63a in the expiration duct 66.

The propelling force for the bag or bellows device 59 is derived from each of the ducts 1 or 3 via the valve 50 and the ducts 47 and 78. The latter duct contains on the one hand a valve 79, on the other hand a double valve 80. The valve 79 is controlled in such a manner that pressure is supplied to the housing 60 during the period when inspiration is required. If for any reason it is desired to achieve this manually, it is possible to do this with the help of a second bubble or bellows device 81 which via the double valve 80 is also connected to the closed housing 60. The valve 80 is adapted so that the duct 78 is automatically disconnected when the bubble or bellows device 81 is subjected to a manual pressure. The bubble or bellows device 81 is inflated with the help of a propellant gas from the duct 78 via a duct 82 with a throttle 83 and distributing valve 84. With the help of the latter valve the device can also be vented via an evacuation line 85.

Between the valves 79 and 80 is provided a control valve 86 shown schematically. With the help of this valve the so-called PEEP pressure is adjusted, that is to say a certain minimum pressure at the end of a patient's expiratory breath. This valve is opened also should the pressure shown on the pressure gauge 77 be at too high a value. The pressure gauge 77, on the other hand, opens the valve 88 through which the closed housing 60 is connected to an evacuation duct 89.

Samples can be taken via the ducts 90 and 91 on the one hand from the patient's expiratory gas, on the other hand from the duct 53 directly after the gasifier 28. The duct 90 is connected via a filter 92 and the duct 91 directly to a change-over valve 93. The samples are conducted then via a further change-over valve 94 to an anesthesia gas meter 95 and an oxygen gas meter 96. With regard to the design of the anaesthesia gas meter reference is made to details given in US-A-4 399 686. Reference is also made to SE-B-428 345 regarding the design and function of the ducts.

A calibration gas can be supplied from a connection duct 97 via the change-over valve 94. The samples are sucked subsequently via a throttle 98 with the help of a pump 99 back into the duct 53.

As a safety measure treatment gas is withdrawn via the duct 100 with check valve 101 from the duct 1 to a tank 102. Should there be a pressure failure in the duct 1, this gas is blown out in a manner known in itself through a whistle 103.

From the duct 1 treatment gas is also withdrawn via the duct 104, a duct 105 and a schematically shown pulse generator 106 to a patient connector 107. This connector may consist, for example of a needle which can be introduced directly into the patient's respiratory passages and to which a pulsating pressure is supplied at a frequency substantially above that customary in normal breathing, e.g. 10 times above normal. This system is used for example when the respiratory passages cannot be reached in any other manner.

The treatment gas may also be conducted from the duct 1 via the duct 104, a filter 108, a valve 109 and a pressure control device 110 via a duct 111 to a point after the gasifier 28. If gas is fed on this route under a strongly reduced pressure with simultaneous pressure measurement the tightness of the ventilator section can be checked. If the pressure does not rise the leakage is equal to or greater than the quantity of gas fed.

Via a further shunt line 112 with a valve 113 and a throttle 114 treatment gas from the duct 1 can be led past the gasifier 28. This duct system is used for rapidly flushing the ventilator section free of anaesthesia gas. The duct with its throttle and valve is, therefore, of a relatively large dimension so that such a rapid flushing can be achieved.

By means of designations 115 and 116 together with a number of schematically shown valves and throttles without designation it is indicated how the treatment gas from the duct 47 can be used on the one hand for the actuation of a patient suction 115, on the other hand for the inflation of a sleeve belonging to a conventional sphygmomanometer 116.

By means of the duct 117 is indicated how the patient's face mask can be adapted to be directly evacuated. Concerning this function reference is made to details given in SE-B-428 757.

Finally reference is made, for example, to US-A-4 421 113 with regard to the function of the ventilator section, though this can be operated, of course, also in some other manner known in itself.

Naturally the invention is not limited only to the embodiment described above but can be varied within the scope of the following claims.

Finally it may be mentioned that the present invention is intended in particular to be used together with the inventions which are described in SE-B-449 052 "Anaesthesia and/or respirator arrangement with multi-purpose utilization of the treatment gas", and SE-B-449 565 "Anaesthesia and/or respirator arrangement with a moistening and/or gasification chamber", submitted at the same time. The content of these patent applications is incorporated, therefore, in the present application.

## Claims

1. Anaesthesia and/or respirator arrangement comprising a gas preparation section which is adapted to prepare a treatment gas of gas from one or more gas sources (7,8,9;7a,8a,9a) and a mechanically operated ventilator section (55-56,58-68) by means of which the prepared gas mixture is supplied to the patient (57), the said ventilator section being adapted so that it also can be operated with the help of a manual reserve system which comprises a manually actuated pump (81) which is adapted to act with the help of a gaseous propellant from the outside upon a movable wall in a chamber (59) containing the treatment gas, the treatment gas and the propellant being completely separated from each other, **characterized** in that the gas from one of the gas sources (7,9;7a) which are utilized for the preparation of the treatment gas is used as the said propellant when the manual pump (81) is activated and also during normal mechanical operation, and by a change-over valve (80) arranged in the duct between the said source (7,9;7a) of the said treatment gas and the said chamber (59) which is adapted to automatically close said duct (78) when the manually actuated pump (81) is acted on to produce a pressure upon the movable wall and to open a duct (82) via the manually actuated pump (81).

2. An arrangement in accordance with claim 1, **characterized** by a reducing valve (49) being arranged between the said source (7,9;7a) of the said treatment gas and the said chamber (59).

3. An arrangement in accordance with claim 1, **characterized** in that the said chamber (59) is constituted of a bag, bellows or the like compressible from the outside which is arranged in a closed rigid chamber (60).

4. An arrangement in accordance with anyone of the preceding claims, **characterized** in that the treatment gas and/or the propellant for the same is in connection with a valve (86) which opens at the maximum permissible pressure.

5. An arrangement in accordance with claim 1, **characterized** in that the said change-over valve (80) is adapted to be reset automatically when the manual operation ceases so that the chamber (59) containing the treatment gas is subjected once again to normal mechanical operation.

6. An arrangement in accordance with claim 5, the ventilator section (55-56,58-68) being adapted to be controlled according to the EMMV method (Extended Mandatory Minute Ventilation), the inspiratory gas supplied to the patient being continuously mesured and integrated and compared with the integral of a corresponding set value, **characterized** in that the manually supplied inspiratory gas too is measured and integrated, the result obtained being added to the integral of the mechanically supplied or spontaneously inspirated treatment gas.

7. An arrangement in accordance with claim 6, **characterized** in that the mechanically supplied gas and the manually supplied gas are both measured by means of the same meter (61,75) downstream of the said change-over valve (80).

## Revendications

1. Dispositif d'anesthésie et/ou de respiration comprenant une section préparation de gaz qui est adaptée pour préparer un gaz de traitement d'un gaz provenant d'une ou plusieurs sources de gaz (7, 8, 9 ; 7a, 8a, 9a) et une section ventilateur fonctionnant mécaniquement (55-56, 58-68) au moyen de laquelle le mélange gazeux préparé est fourni au malade (57), ladite section ventilateur étant adaptée de manière qu'on puisse également l'actionner à l'aide d'un système de réserve manuel qui comprend une pompe actionnée à la main (81) qui est adaptée pour agir à l'aide d'un vecteur gazeux de l'extérieur sur une paroi mobile dans une chambre (59) comprenant le gaz de traitement, le gaz de traitement et le vecteur étant complètement séparés l'un de l'autre, caractérisé en ce que le gaz de l'une des sources de gaz (7, 9 ; 7a) qui sont utilisées pour la préparation du gaz de traitement, est utilisé comme vecteur lorsque la pompe manuelle (81) est activée et également pendant le fonctionnement mécanique normal, et par une vanne à commutateur (80) disposée dans le conduit entre ladite source (7, 9 ; 7a) dudit gaz de traitement et ladite chambre (59) qui est adaptée pour fermer automatiquement ledit conduit (78) lorsque la pompe actionnée à la main (81) est actionnée pour produire une pression sur la paroi mobile et pour ouvrir un conduit (82) via la pompe actionnée à la main (81).

2. Dispositif selon la revendication 1, caractérisé en ce qu'un détendeur (49) est disposé entre ladite source (7, 9 ; 7a) dudit gaz de traitement et ladite chambre (59).

3. Dispositif selon la revendication 1, caractérisé en ce que ladite chambre (59) est constituée d'un sac, d'un soufflet, etc, compressible à partir de l'extérieur, qui est disposé dans une chambre rigide fermée (60).

4. Dispositif selon l'une quelconque des revendications précédentes, caractérisé en ce que le gaz de traitement et/ou son vecteur est en liaison avec une vanne (86) qui s'ouvre à la pression maximale permissible.

5. Dispositif selon la revendication 1, caractérisé en ce que ladite vanne à commutateur (80) est adaptée pour être remise en place automatiquement lorsque l'opération manuelle cesse de manière que la chambre (59) contenant le gaz de traitement soit encore soumise à un fonctionnement mécanique normal.

6. Dispositif selon la revendication 5, la section ventilateur (55-56, 58-68) étant adaptée pour être commandée selon le procédé EMMV ("Extended Mandatory Minute Ventilation" - Ventilation Minute Obligatoire Etendue), le gaz d'aspiration fourni au malade étant continuellement mesuré, intégré et comparé avec l'intégrale d'une valeur fixée correspondante, caractérisé en ce que le gaz d'inspiration fourni à la main est également mesuré et intégré, le résultat obtenu étant ajouté à l'intégrale du gaz de traitement fourni mécaniquement ou inspiré spontanément.

7. Dispositif selon la revendication 6, caractérisé en ce que le gaz fourni mécaniquement et le gaz fourni manuellement sont tous deux mesurés au moyen du même appareil en aval de ladite vanne à commutateur (80).

**Patentansprüche**

1. Narkose- und/oder Beatmungsanordnung mit einem Gaszubereitungsabschnitt, welcher für das Zubereiten eines Behandlungsgases aus Gas von einer oder mehreren Gasquellen (7, 8, 9; 7a, 8a, 9a) ausgelegt ist und mit einem maschinell betätigten Ventilatorabschnitt (55-56, 58-68), durch welchen die zubereitete Gasmischung dem Patienten (57) zugeführt wird, wobei der Ventilatorabschnitt so ausgelegt ist, daß er auch mit Hilfe eines manuellen Reservesystems betätigt werden kann, der eine handbetätigte Pumpe (81) aufweist, die dafür ausgelegt ist, mit Hilfe eines gasförmigen Treibmittels von der Außenseite her auf eine bewegbare Wand in einer Kammer (59) einzuwirken, die das Behandlungsgas enthält, wobei das Behandlungsgas und das Treibmittel vollständig voneinander getrennt sind, dadurch gekennzeichnet, daß das Gas von einer der Gasquellen (7, 9; 7a), welche für die Zubereitung des Behandlungsgases verwendet werden, als das Treibmittel verwendet wird, wenn die Handpumpe (81) betätigt wird und ebenso während der normalen maschinellen Betätigung, und daß ein Umschaltventil (80) in der Leitung zwischen der Quelle (7, 9; 7a) des Behandlungsgases und der Kammer (59) angeordnet ist, welches so ausgelegt ist, daß es automatisch die Leitung (78) abtrennt, wenn auf die handbetätigte Pumpe (81) eingewirkt wird, um einen Druck auf die bewegbare Wand auszuüben, und um über die handbetätigte Pumpe (81) eine Leitung (82) zu öffnen.

2. Anordnung nach Anspruch 1, gekennzeichnet durch ein Reduzierventil (49), welches zwischen der Quelle (7, 9; 7a) des Behandlungsgases und der Kammer (59) angeordnet ist.

3. Anordnung nach Anspruch 1, dadurch gekennzeichnet, daß die Kammer (59) aus einem Sack, Balg oder dergleichen besteht, welcher von der Außenseite her zusammendrückbar und in einer geschlossenen festen Kammer (60) angeordnet ist.

4. Anordnung nach einem der vorstehenden Ansprüche, dadurch gekennzeichnet, daß das Behandlungsgas und/oder das Treibmittel für dasselbe mit einem Ventil (86) in Verbindung steht, welches bei dem maximal zulässigen Druck öffent.

5. Anordnung nach Anspruch 1, dadurch gekennzeichnet, daß das Umschaltventil (80) so ausgelegt ist, daß es automatisch zurückschaltet, wenn die Handbetätigung aufhört, so daß die Kammer (59), welche das Behandlungsgas enthält, wieder der normalen maschinellen Betätigung ausgesetzt ist.

6. Anordnung nach Anspruch 5, wobei der Ventilatorabschnitt (55-56, 58-68) so ausgelegt ist, daß er nach dem EMMV-Verfahren (Extended Mandatory Minute Ventilation) gesteuert werden kann, wobei das dem Patienten zugeführte Beatmungsgas kontinuierlich gemessen und integriert und mit dem Integral eines entsprechenden Sollwertes verglichen wird, dadurch gekennzeichnet, daß das von Hand zugeführte Beatmungsgas ebenfalls gemessen und integriert wird, wobei das erhaltene Ergebnis dem Integral des maschinell zugeführten oder spontan eingeatmeten Behandlungsgases hinzuaddiert wird.

7. Anordnung nach Anspruch 6, dadurch gekennzeichnet, daß das maschinell zugeführte Gas und das von Hand zugeführte Gas beides mit Hilfe desselben Meßgerätes (61, 65) stromabwärts von dem Umschaltventil (80) gemessen wird.

*Fig.1A*

EP 0 166 305 B1

*Fig.1B*